# EUROPEAN PATENT APPLICATION

(11) **EP 4 545 956 A1**
(43) Date of publication of application: **30.04.2025**
(21) Application number: 23826797.5
(22) Date of filing: 25.04.2023
(51) Int. Cl.: G01N 27/00, G01N 33/543

(54) **METHODS OF MEASUREMENT AND ANALYSIS FOR DETECTION AND QUANTIFICATION OF PATHOGENS, MICROORGANISMS OR PROTEINS, AND COMPUTER PROGRAM FOR IMPLEMENTING SAID METHODS**

(30) Priority: 24.06.2022 JP 2022101989
(71) Applicant: Aipore Inc., Tokyo, 150-8512 (JP)
(72) Inventor: NAONO, Norihiko, Tokyo 150-8512 (JP); SAKAMOTO,Osamu, Tokyo 150-8512 (JP); TAKEI,Hiroyasu, Tokyo 150-8512 (JP)
(74) Representative: Mewburn Ellis LLP
(86) International application number: PCT/JP2023/016346
(87) International publication number: WO 2023/248608

(57) **Abstract**

A method for preparing a data for estimating a concentration of an unknown antigen in an unknown sample in which a concentration of a target antigen is unknown; the method using a sensor including two chambers separated by a partition wall and communicating with each other through a pore, and an electrode in each of the two chambers; the method including:
a step of preparing a first sensor including two chambers configured to be electrically connected via a first pore and an electrolyte, and placing a known measurement target sample, which includes a known sample containing the target antigen with a known antigen concentration and an antibody-modified particle whose surface is modified with an antibody that specifically binds to the target antigen, into one of the chambers of the first sensor;
a step of applying a voltage between the electrodes of each chamber of the first sensor to cause an ionic current to flow between the two electrodes of the first sensor via the first pore, and measuring a transient change in the ionic current caused by the antibody-modified particles in the known measurement target sample passing through the first pore as a known pulse waveform group consisting of a plurality of known pulse waveforms;
a step of calculating a known pulse waveform feature that expresses a shape characteristic for each of the plurality of known pulse waveforms belonging to the known pulse waveform group;
a step of calculating a known distribution feature that expresses a distribution characteristic of the known pulse waveform feature within the known pulse waveform group; and
a step of training an Al model using the known distribution feature as a training data and the known antigen concentration as a training label to create a trained Al model.

## Description

### FIELD OF THE INVENTION

The present invention relates to a measurement and analysis method for detecting and quantifying pathogens, microorganisms, or proteins contained in a sample, and a computer program for performing said method.

### BACKGROUND OF THE INVENTION

Detection and quantification of minute amounts of proteins, microorganisms, and pathogens contained in biological particles is one of the most fundamental methods for identifying or inferring the cause of disease. In the detection and quantification of proteins, a so-called immunoassay is widely used, in which the protein to be detected or quantified is used as an antigen and an antibody that specifically binds to the antigen is used. For example, turbidimetry, absorbance method, immunochromatography, and the like, which measure changes in light absorption or reflection due to the aggregation phenomenon of such antibody-modified microparticles, or alternatively, ELISA (Enzyme-Linked Immuno Sorbent Assay), CLEIA (Chemiluminescent Enzyme Immunoassay), and CLIA (Chemiluminescent Immunoassay), which label antibodies or antigens with chemiluminescent substances to measure the absorbance or luminescence, are widely used. In recent years, in order to increase the sensitivity of such immunoassays, a so-called digital ELISA has been proposed in which antibody-modified particles in minute wells are individually measured (Patent Literature 1).

In the detection and quantification of microorganisms, in addition to immunoassays for detecting and quantifying antibodies specific to the target microorganism and proteins that constitute the target microorganism, the PCR (primer chain reaction) method for detecting and quantifying microbial genes is also widely used (Patent Literature 2).

In these circumstances, a technology has been proposed that aims to achieve both the above-mentioned high sensitivity and low cost by directly measuring pathogens (Non-Patent Literature 1) and proteins (Non-Patent Literature 2) using the so-called pore electrical resistance method (Patent Literatures 3 and 4), in which nano-sized particles in an electrolyte are driven by electrophoresis and the transient change in electrical resistance is observed as the particles pass through pores close to the size of the particles.

### PRIOR ART

### Patent Literature

[Patent Literature 1] U.S. Patent No. 9,482,662
[Patent Literature 2] U.S. Patent No. 4,683,195
[Patent Literature 3] U.S. Patent No. 9,726,636
[Patent Literature 4] Japanese Patent No. 6,719,773

### Non-Patent Literature

[Non-Patent Literature 1] Taniguchi, Masateru, et al. "Combining machine learning and nanopore construction creates an artificial intelligence nanopore for coronavirus detection." Nature communications 12.1 (2021): 1-8.
[Non-Patent Literature 2] Yusko, Erik C., et al. "Real-time shape approximation and fingerprinting of single proteins using a nanopore." Nature nanotechnology 12.4 (2017): 360-367.

### SUMMARY OF THE INVENTION

However, each of the traditional immunoassay methods has its own limitations. For example, traditional methods that optically monitor antibody-modified aggregation, such as turbidimetry and immunochromatography, suffer from a lack of sensitivity when measuring samples with dilute amounts of the target protein or pathogen. Immunoassays using chemiluminescent substances such as ELISA can improve sensitivity compared to turbidimetric methods, but have problems such as high costs and complicated measurement procedures. Furthermore, although the PCR method for analyzing the genetic information of microorganisms is highly sensitive, it has been similarly difficult to achieve both high sensitivity and reduced costs and labor.

Conventional methods that use pore electrical resistance directly measure target particles (microorganisms, proteins, or the like) one by one. In addition, the method uses a simple configuration that combines a sensor device with a pore and an amplifier, and has the advantage that it does not require complex optical systems or measurement protocols, especially in applications that require highly sensitive detection and quantification.

However, the way particles pass through the pores has a statistical distribution. For example, even if the microorganisms being measured are of the same species, their shapes are not necessarily the same, and the signals obtained from each microorganism vary. Further, for example, because proteins are small compared to the diameter of the pores, the measured signal differs depending on whether they pass near the center or the edge of the pore, which also causes variation in the measured signal. Then, such signal variations can cause errors in the analysis of the measurement results of the pore electrical resistance method, leading to reduced accuracy in the detection and quantification of these microorganisms and proteins.

The present invention has been made in view of the above circumstances, and provides a method for enabling highly sensitive and highly accurate detection or quantification of proteins, microorganisms, or pathogens by analyzing measurement data obtained by a pore electrical resistance method. That is, the present invention can provide the following aspects.

### [Aspect 1]

A method for preparing a data for estimating a concentration of an unknown antigen in an unknown sample in which a concentration of a target antigen is unknown; the method using a sensor comprising two chambers separated by a partition wall and communicating with each other through a pore, and an electrode in each of the two chambers; the method comprising:
a step of preparing a first sensor comprising two chambers configured to be electrically connected via a first pore and an electrolyte, and placing a known measurement target sample, which includes a known sample containing the target antigen with a known antigen concentration and an antibody-modified particle whose surface is modified with an antibody that specifically binds to the target antigen, into one of the chambers of the first sensor;
a step of applying a voltage between the electrodes of each chamber of the first sensor to cause an ionic current to flow between the two electrodes of the first sensor via the first pore, and measuring a transient change in the ionic current caused by the antibody-modified particles in the known measurement target sample passing through the first pore as a known pulse waveform group consisting of a plurality of known pulse waveforms;
a step of calculating a known pulse waveform feature that expresses a shape characteristic for each of the plurality of known pulse waveforms belonging to the known pulse waveform group;
a step of calculating a known distribution feature that expresses a distribution characteristic of the known pulse waveform feature within the known pulse waveform group; and
a step of training an Al model using the known distribution feature as a training data and the known antigen concentration as a training label to create a trained Al model.

### [Aspect 2]

The method according to aspect 1, wherein the method further comprises:
a step of preparing a second sensor having two chambers configured to be electrically connected via a second pore and an electrolyte, and placing unknown measurement target sample, which includes an unknown sample containing the target antigen with an unknown concentration and the antibody-modified particles, into one of the chambers of the second sensor;
a step of applying a voltage between the electrodes of each chamber of the second sensor to cause an ionic current to flow between the two electrodes of the second sensor via the second pore, and measuring a transient change in the ionic current caused by the antibody-modified particles in the unknown measurement target sample passing through the second pore as an unknown pulse waveform group consisting of a plurality of unknown pulse waveforms;
a step of calculating an unknown pulse waveform feature that expresses a shape characteristic for each of the unknown pulse waveforms belonging to the unknown pulse waveform group;
a step of calculating an unknown distribution feature that expresses a distribution characteristic of the unknown pulse waveform feature within the unknown pulse waveform group; and
a step of inputting the unknown distribution feature into the trained Al model, and causing the trained Al model to output an estimate of the unknown antigen concentration.

### [Aspect 3]

The method according to aspect 1 or 2, wherein the method further comprises a step of:
calculating a known branch distribution feature from the known pulse waveform feature belonging to the known pulse waveform group; and
if the known branch distribution feature satisfies a branch condition, training a first Al using the known distribution feature as a training data and the known antigen concentration as a training label to create a first trained Al, or
if the known branch distribution feature does not satisfy the branch condition, training a second AI using the known distribution feature as a training data and the known antigen concentration as a training label to create a second trained Al.

### [Aspect 4]

The method according to aspect 3, wherein the method further comprises:
a step of preparing a second sensor comprising two chambers configured to be electrically connected via a second pore and an electrolyte, and placing an unknown measurement target sample, which includes an unknown sample containing the target antigen with unknown concentration and the antibody-modified particles, into one of the chambers of the second sensor;
a step of applying a voltage between the electrodes of each chamber of the second sensor to cause an ionic current to flow between the two electrodes of the second sensor via the second pore, and measuring a transient change in the ionic current caused by the antibody-modified particles in the unknown measurement target sample passing through the second pore as an unknown pulse waveform group consisting of a plurality of unknown pulse waveforms;
a step of calculating an unknown pulse waveform feature that expresses a shape characteristic for each of the unknown pulse waveforms belonging to the unknown pulse waveform group;
a step of calculating an unknown branch distribution feature that expresses a distribution characteristic of the unknown pulse waveform feature within the unknown pulse waveform group; and
a step of calculating an unknown branch distribution feature from the unknown pulse waveform feature belonging to the unknown pulse waveform group, and
if the unknown branch distribution feature satisfies the distribution condition, inputting the unknown distribution feature to the first trained Al, and outputting an estimated value of the unknown antigen concentration, or
if the unknown branch distribution feature does not satisfy the distribution condition, inputting the unknown distribution feature to the second trained Al and outputting an estimate of the unknown antigen concentration.

### [Aspect 5]

The method according to any one of aspects 1 to 4, wherein the method further comprises a step of:
if the known antigen concentration satisfies a branch condition, training a first Al using the known distribution feature as a training data and the known antigen concentration as a training label to create a first trained Al, or
If the known antigen concentration does not satisfy the branch condition, training a second AI using the known distribution feature as a training data and the known antigen concentration as a training label to create a second trained Al.

### [Aspect 6]

A method for preparing a data to estimate whether a target microparticle is present in an unknown sample, the method using a sensor comprising two chambers separated by a partition wall and communicating with each other through a pore, and an electrode in each of the two chambers; the method comprising:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor;
a step of applying a voltage between electrodes of each of the chambers of the first sensor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms;
a step of calculating a known pulse waveform feature that expresses a shape characteristic for each of the plurality of known pulse waveforms;
a step of calculating a known distribution feature that expresses a distribution characteristic of the known pulse waveform feature within the known pulse waveform group; and
a step of training an Al model using the distribution feature as a training data and information expressing the presence or absence of the target particle in the known sample as a training label to create a trained Al model.

### [Aspect 7]

The method according to aspect 6, wherein the method further comprises:
a step of preparing a second sensor having two chambers configured to be electrically connected via a second pore and an electrolyte, and placing an unknown measurement target sample, which includes an unknown sample in which the presence or absence of the target microparticle is unknown and an electrolyte, into one of the chambers of the second sensor;
a step of applying a voltage between electrodes of each of the chambers of the second sensor;
a step of measuring a transient change in an ion current caused by a microparticle contained in the unknown measurement target sample passing through the second pore while an ion current is caused to flow between two electrodes of the second sensor via the second pore, as an unknown pulse waveform group belonging to the unknown measurement target sample, the unknown pulse waveform group consisting of a plurality of unknown pulse waveforms;
a step of calculating an unknown pulse waveform feature that expresses a shape characteristic for each of the plurality of unknown pulse waveforms;
a step of calculating an unknown distribution feature that expresses a distribution characteristic of the unknown pulse waveform feature within the unknown pulse waveform group; and
a step of inputting the unknown distribution feature into the trained Al model to estimate whether the target microparticle is contained in the unknown sample.

### [Aspect 8]

The method according to any one of aspects 1 to 7 or 10, or the program according to any one of aspects 12 to 14 as below, wherein the target microparticle is a bacteria.

### [Aspect 9]

The method according to any one of aspects 1 to 7 or 10, or the program according to any one of aspects 12 to 14 as below, wherein the target microparticle is a virus.

### [Aspect 10]

A method for preparing a data to estimate whether a target microparticle is present in an unknown sample, the method using a sensor comprising two chambers separated by a partition wall and communicating with each other through a pore, and an electrode in each of the two chambers; the method comprising:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor;
a step of applying a voltage between electrodes of each of the chambers of the first sensor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms;
a step of calculating a known pulse waveform feature that expresses a shape characteristic for each of the known pulse waveforms; and
a step of calculating a known distribution feature that expresses a distribution characteristic of the known pulse waveform feature within the known pulse waveform group; and
a step of training an Al model using the known pulse waveform feature and the known distribution feature as training data and information expressing the presence or absence of the target particle in the known sample as training labels to create a trained Al model.

### [Aspect 11]

The method according to any one of aspects 1 to 10, or the program according to any one of aspects 12 to 14 as below, wherein the known pulse waveform feature is calculated from an output of a convolutional neural network.

### [Aspect 12]

A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a first sensor comprising two chambers configured to be electrically connected via a first pore and an electrolyte, and placing a known measurement target sample, which includes a known sample containing the target antigen with a known antigen concentration and an antibody-modified particle whose surface is modified with an antibody that specifically binds to the target antigen, into one of the chambers of the first sensor, by the processor;
a step of applying a voltage between the electrodes of each chamber of the first sensor to cause an ionic current to flow between the two electrodes of the first sensor via the first pore, and measuring a transient change in the ionic current caused by the antibody-modified particles in the known measurement target sample passing through the first pore as a known pulse waveform group consisting of a plurality of known pulse waveforms, by the processor;
a step of calculating a known pulse waveform feature that expresses a shape characteristic for each of the plurality of known pulse waveforms belonging to the known pulse waveform group, by the processor;
a step of calculating a known distribution feature that expresses a distribution characteristic of the known pulse waveform feature within the known pulse waveform group, by the processor; and
a step of training an Al model using the known distribution feature as a training data and the known antigen concentration as a training label to create a trained Al model, by the processor.

### [Aspect 13]

A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor, by the processor;
a step of applying a voltage between electrodes of each of the chambers of the first sensor, by the processor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms, by the processor;
a step of calculating a known pulse waveform feature that expresses a shape characteristic for each of the plurality of known pulse waveforms, by the processor;
a step of calculating a known distribution feature that expresses a distribution characteristic of the known pulse waveform feature within the known pulse waveform group, by the processor; and
a step of training an Al model using the distribution feature as a training data and information expressing the presence or absence of the target particle in the known sample as a training label to create a trained Al model, by the processor.

### [Aspect 14]

A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor, by the processor;
a step of applying a voltage between electrodes of each of the chambers of the first sensor, by the processor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms, by the processor;
a step of calculating a known pulse waveform feature that expresses a shape characteristic for each of the known pulse waveforms, by the processor; and
a step of calculating a known distribution feature that expresses a distribution characteristic of the known pulse waveform feature within the known pulse waveform group, by the processor; and
a step of training an Al model using the known pulse waveform feature and the known distribution feature as training data and information expressing the presence or absence of the target particle in the known sample as training labels to create a trained Al model, by the processor.

### [Aspect 15]

The method according to any one of aspects 1 to 10, or the program according to any one of aspects 12 to 14, wherein the antigen is replaced with an antibody and the antibody is replaced with an antigen.

According to the present invention, it is possible to obtain an effect that it is possible to detect or quantify proteins, microorganisms, and pathogens with high sensitivity and high accuracy by analyzing measurement data obtained by the pore electrical resistance method.

### BRIEF DESCRIPTION OF THE DRAWINGS

FIG. 1 shows an example of the cross-sectional structure of a sensor used in the present invention.
FIG. 2 shows an example of a pulse-like transient change in an ion current flowing between electrodes.
FIG. 3 shows an example of a pulse feature that can be used in the present invention.
FIG. 4 shows a schematic representation of a chamber filled with a measurement target sample.
FIG. 5 shows the results of measuring a pulse signal as shown in FIG. 2 using the sensor of FIG. 1, using prostate-specific antigen (PSA) as the quantification target protein (target antigen) and 200 nm-diameter latex beads modified with anti-PSA antibodies.
FIG. 6 shows the frequency distribution of pulse waveform feature b (peak current) when a measurement target sample NTC containing no PSA, and measurement target samples containing PSA of 300 fg/mL, 3 pg/mL, 30 pg/mL, 300 pg/mL, and 3 ng/mL are measured sequentially, using the method of the present invention.
FIG. 7 shows an example of a distribution feature for pulse feature b.
FIG. 8 is a diagram for explaining Al training using pulse feature and distribution feature according to the present invention.
FIG. 9 is a diagram showing the PSA concentration dependence of the distribution feature of the pulse feature b (pulse peak current) shown in FIG. 6.
FIG. 10 is a diagram showing the dependence of the number of pulses n per pulse group on the PSA concentration for the measurement shown in FIG. 6.
FIG. 11 is a diagram showing the correlation of the pulse number ratio r_{b} with a peak current of 4.5 nA or more, which is another distribution feature, with the PSA concentration for the measurement shown in FIG. 6.
FIG. 12 is a chart explaining a method for training an Al model according to an embodiment of the present invention.
FIG. 13 is a confusion matrix obtained when measuring 60 unknown samples each of Staphylococcus aureus and Staphylococcus epidermidis, which are bacteria with similar morphology, and classifying the results using a trained Al according to the prior art and a trained Al according to the present invention.

### DETAILED DESCRIPTION OF THE INVENTION

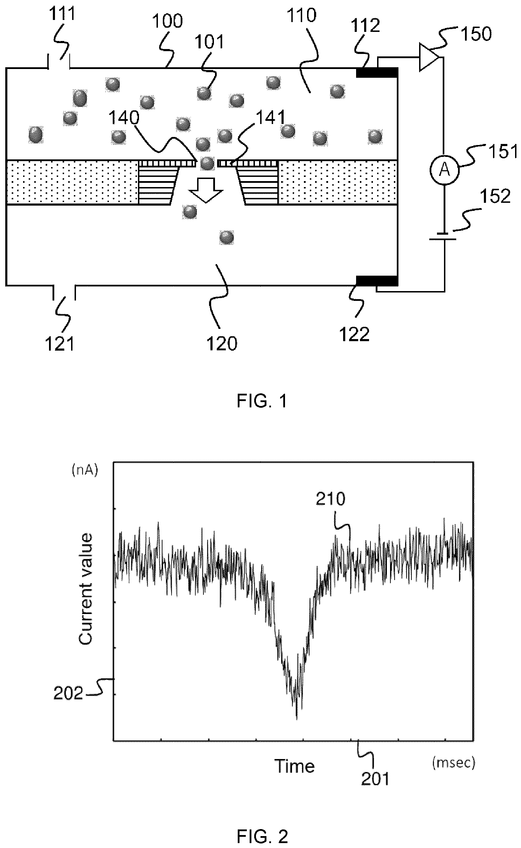

In the present invention, a pulse waveform is measured by passing fine particles, such as microorganisms such as bacteria and viruses, which are the subject of detection and quantification, through a pore in a sensor as shown in FIG. 1. In addition, in the present invention, a pulse waveform is measured by passing antibody-modified particles, the surface of which is modified with an antibody that specifically binds to the protein to be detected or quantified, through a pore in a sensor such as that shown in FIG. 1. Further, these pulse waveforms are then analyzed by the method of the present invention to detect and quantify target microparticles such as pathogens and proteins. It should be noted that the procedures and principles of the present invention are the same when detecting or quantifying target antigens using antibody-modified particles and when detecting or quantifying target antibodies using antigen-modified particles. In order to avoid unnecessarily lengthy description, the following will explain the former embodiment, that is, the detection or quantification of a target antigen using antibody-modified particles.

In addition, in another embodiment of the present invention, antibodies can be detected and quantified in biological samples. That is, this other embodiment can be understood by replacing the antigen with the antibody and the antibody with the antigen in the previous and following explanations. Although the explanation will not be repeated below, please note that each description in this specification can be appropriately replaced as described above as long as there is no contradiction.

An example of the cross-sectional structure of a sensor used in the present invention is shown in FIG. 1. The sensor 100 has a cross-sectional structure in which two chambers 110 and 120 are separated by a partition wall 141 and connected via a pore 140 provided in the partition wall 141. The two chambers are respectively provided with electrodes 112 and 122. A sample containing measurement target particles suspended in an electrolyte is introduced into the chamber 110 via an inlet 111, and the electrolyte is introduced into chamber 120 via an inlet 121. A voltage is applied to the two electrodes by voltage source 152. For example, when a voltage is applied between the electrodes 112 and 122, an ionic current flows through the pore. In addition, as used herein, the term "chamber" refers to a portion capable of storing a sample solution (electrolyte). In addition, as used herein, an electrode is defined as being "inside a chamber" if it is in a position where it can come into contact with the sample liquid in the chamber (that is, where electricity can be passed therethrough). In addition, as used herein, "filling" a chamber does not necessarily mean filling the entire volume of the chamber, but may leave some void as long as the sensor functions. That is, "filling" may be considered interchangeably herein with "injecting" or "introducing." For example, as illustrated in FIG. 1, when a particle present in chamber 110 passes through the pore 140, the ionic current is temporarily interrupted and then returns after the particle passes through chamber 120. Therefore, every time a particle passes through the pore140, the ion current flowing between the electrodes in FIG. 1 exhibits a pulse-like transient change as shown in FIG. 2. In the example of FIG. 1, this is measured by an ammeter 151. It should be noted that FIG. 1 is only one example of a sensor used in the present invention, and any form of sensor may be used as long as it has a structure in which two chambers, each having an electrode, communicate with each other through a pore.

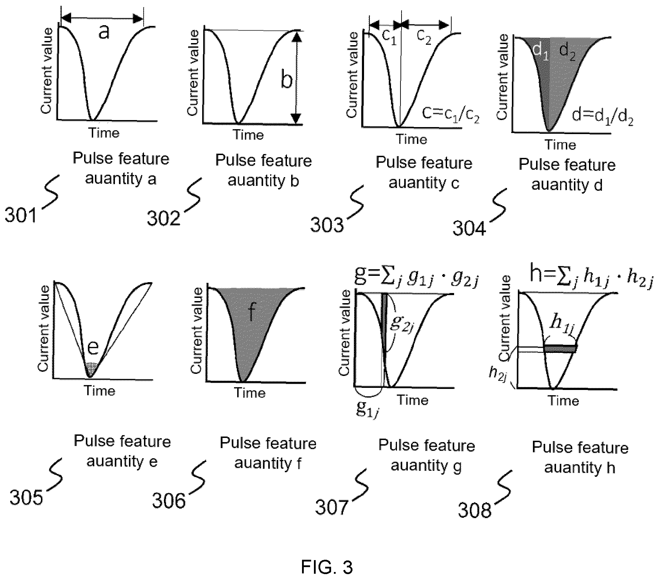

FIG. 2 shows an example 210 of a pulse waveform of a current caused by the passage of one particle. The pulse waveform is a current value 202 at each time 201. The vertical axis 202 may be a voltage value. The shape of the pulse signal changes depending on the size, shape, and surface condition of the passing particle. In the present invention, a pulse feature that expresses the pulse shape is calculated from the pulse waveform measurement result of one passing particle. FIG. 3 show examples of pulse feature used in the present invention. For example, pulse feature a 301 is a pulse width, and pulse feature b 302 is a peak current value of a pulse. FIG. 3 shows an example of pulse feature, and any feature that expresses the shape of a pulse may be used. Further, for example, the output of a convolutional layer in a CNN (Convolutional Neural Network) may also be a feature that expresses the shape of a pulse in the present invention. For example, in a CNN, the feature z i (I) of the I^{th} layer when the time in the pulse is i may be calculated as follows:
${z}_{i}^{\left(l\right)} = f \left({u}_{i}^{\left(l\right)}\right) = f \left({\sum }_{p = 0}^{H - 1} {z}_{Si + p}^{\left(l-1\right)} {h}_{p} + bi\right)$ wherein, H is the filter size, S is the stride, hₚ is the filter coefficient at p, p is the position within the filter, and b is the bias.

The present invention can be used to analyze pulse waveforms generated when biological particles such as bacteria, viruses, and exosomes pass through pores, or when antibody-modified particles pass through the pore. In the following, the problems with the conventional technology and the principles of the present invention will be described using the latter as an example. Hereinafter, a protein to be detected or quantified is referred to as a target antigen. In addition, as used herein, a known sample containing a target antigen with a known antigen concentration and an antibody-modified particle whose surface is modified with an antibody that specifically binds to the target antigen may be referred to as a "known measurement target sample." Further, an unknown sample containing a target antigen with an unknown antigen concentration and an antibody-modified particle whose surface is modified with an antibody that specifically binds to the target antigen may be referred to as an "unknown measurement target sample." These measurement target samples can be mixed and prepared according to the reaction efficiency between the target antigen and the antibody. It may be understood that these measurement target samples include an electrolyte for providing electrical continuity between the chambers. The electrolytes injected into both chambers may have different compositions or may have the same composition. It should be noted that any electrolyte known in the art may be used depending on the application of the present invention.

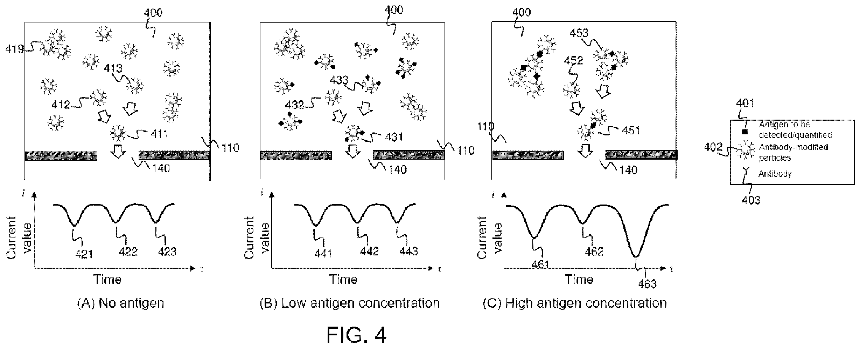

FIGS. 4A to 4C are schematic diagrams showing the state in which the chamber 110 is filled with the sample to be measured. The legend in FIG. 4 shows a target antigen 401, an antibody-modified particle 402, and an antibody 403. When a known sample or an unknown sample does not contain a target antigen, as shown in FIG. 4(a), the target antigen bound to the target antibody is not present in the measurement target sample. When a voltage is applied between the electrodes 112 and 122 in this state, the antibody-modified particles in the measurement target sample each pass through the pore 140 individually, as shown in FIG. 4A. In the example of FIG. 4A, the pulse signals generated when antibody-modified particles 411, 412, and 413 pass through the pore 140 are 421, 422, and 423, respectively. If the measurement target sample does not contain the target antibody, the antibody-modified particles will not bind through the target antigen. Therefore, most of the antibody-modified particles pass through the pore alone.

When a target antigen is present in the measurement target sample, the target antigen is loaded into chamber 110 in a state in which it is bound to antibody-modified particles, as shown in FIG. 4B and FIG. 4C. FIG. 4B shows a case where the concentration of the target antigen is low. Although the target antigen is bound to the antibody-modified particle, no aggregation of the antibody-modified particles occurs via the target antigen, unlike FIG. 4C, which will be described below.

On the other hand, as shown in FIG. 4C, when the concentration of the target antigen is high, the antibody-modified particles form aggregates via the target antigen. The waveform of the pulse signal generated when these pass through the pore has a peak current value greater than that of the pulse signal generated when the antibody-modified particle passes through the pore alone. This is because when a large particle passes through a pore, the ionic current through the pore is impeded to a greater extent than when a small particle passes through the pore. For example, in FIG. 4C, the pulse signals of agglomerate 451, particle 452 and agglomerate 453 passing through pore 140 are 461, 462 and 463, respectively. This is the reason why the peak current value of the pulse waveform of agglomerate 451 is greater than that of single particle 452, and why the peak current value of the pulse waveform of agglomerate 453 is even greater. Therefore, for a target antigen concentration such as that shown in FIG. C, by analyzing a pulse feature, for example, the peak current value, it is possible to estimate the aggregation state of the antibody-modified particles in the sample to be measured, and from that, the presence or absence and concentration of the target antigen in the sample to be measured can be estimated.

As described above, in the present invention, the adhesion of the target protein, which is an antigen, to the antibody-modified beads (FIG. 4) or the state of aggregation between antibody-bound beads via the target protein is observed by the sensor shown in FIG. 1. However, in real samples, the binding of antigens to antibodies is a stochastic phenomenon, and non-specific adsorption between beads that is not dependent on the antigen-antibody reaction also occurs. For this reason, the measurement target sample generally contains a mixture of single antibody-modified beads to which the target protein is not attached, single antibody-modified beads to which the target protein is attached, and beads that have aggregated due to non-specific adsorption or the like. In addition, the pulse waveforms generated by the passage of a particle through a sensor pore as shown in FIG. 1 have a statistical distribution even if they are caused by the same particle. For example, the shape of the pulse waveform will be different when the particle passes through the center of the pore and when it passes through the edge.

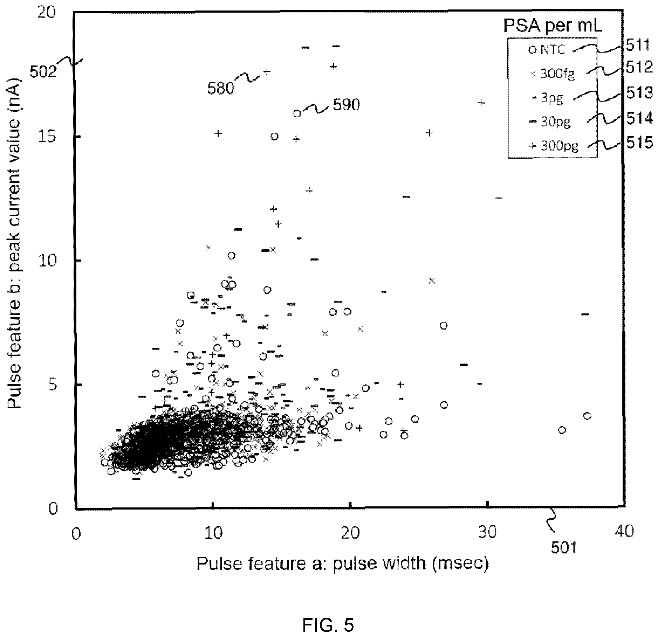

For example, FIG. 5 shows the result of measuring a pulse signal such as that illustrated in FIG. 2 using the sensor of FIG. 1, with prostate-specific antigen (PSA) as the quantified protein (target antigen) and 200 nm diameter latex beads modified with anti-PSA antibodies. The horizontal axis 501 represents the pulse width (pulse feature a shown in FIG. 3), and the vertical axis 502 represents the peak current value (pulse feature b), with one pulse plotted as one point. Furthermore, the plot 511 for NTC (No Template Control) is a measurement target sample that does not contain PSA, the plot 512 for 300 fg is a measurement target sample that contains PSA at a concentration of 300 fg/mL, and so on for the plots of 3 pg, 30 pg, and 300 pg. In general, when larger aggregates pass through a pore, both the pulse peak current and pulse height are greater than when a single bead passes through the pore. As can be seen from FIG. 5, in general, there is a tendency for both the pulse height and pulse width to increase as the concentration of PSA, the protein to be measured, increases; however, even in the region where the pulse height is 15 nA or more, there is a plot 590 of NTC and a plot 580 of 300 pg/mL. Since the sample measured by NTC does not contain PSA, pulses such as plot 590 will vary due to nonspecific adsorption between beads or variations in the pore passage paths of the antibody-modified particles, and will have a certain statistical distribution. For this reason, as disclosed in Patent Literature 4, even if multiple pulse features, such as those illustrated in FIG. 3, are analyzed for one pulse, this does not necessarily reflect the tendency of multiple pulse waveforms obtained from a sample.

A conventional method has been disclosed in which a sensor such as that shown in FIG. 1 is used to train an Al using the characteristics of the pulse waveform resulting from the passage of particles through a pore as training data to classify and analyze particles (see Patent Literature 4). In this conventional method, the Al is trained on the characteristics of the pulse waveform shape, for example, as shown in FIG. 3, for each pulse. However, when training a phenomenon in which the shapes of multiple pulses with the same teacher label vary greatly, as in FIG. 5, the performance significantly degrades, which was an issue that could not be resolved.

In the present invention, therefore, a plurality of pulse waveforms are measured from one measurement target sample, and the plurality of pulse waveforms obtained from one measurement target sample are called one pulse waveform group. In addition, as shown in FIG. 3, in addition to the pulse feature that expresses the characteristics of the shape of a single pulse, a feature that expresses the distribution of pulse features within a single pulse waveform group is called a distribution feature.

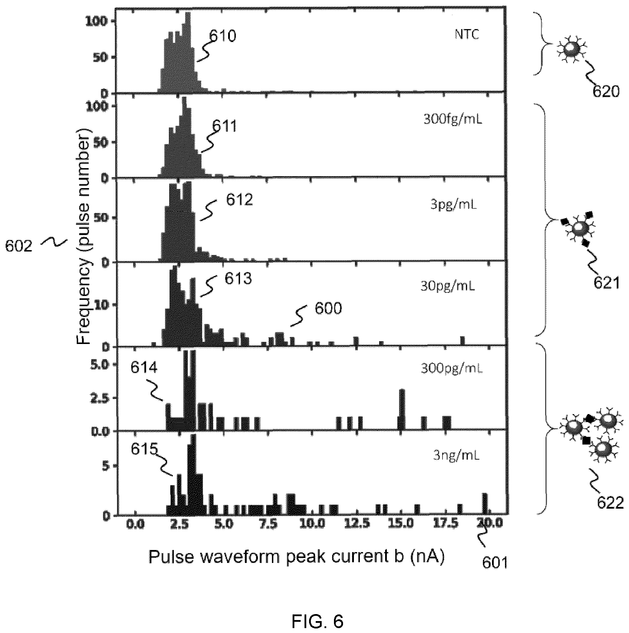

FIG. 6 shows the frequency distribution (610 to 615 in FIG. 6) of pulse waveform feature b (peak current) when measurement target samples containing PSA at levels ranging from a measurement target sample NTC containing no PSA, and measurement target samples containing PSA of 300 fg/mL, 3 pg/mL, 30 pg/mL, 300 pg/mL, and 3 ng/mL are measured sequentially, using the method of the present invention. Since a change is observed in the distribution of peak current b, which is a pulse feature, with respect to a change in PSA concentration per mL, it is understood that this can be utilized to quantify the target antigen, PSA. In measurements where the PSA concentration is higher than 30 pg/mL, pulses 600 with a large peak current b of 5 nA or more appear, and the higher the PSA concentration, the more numerous they become. This reflects the formation of aggregates of antibody-modified particles as shown in FIG. 4C. FIG. 6 shows the measurement results of a known sample with a known target antigen concentration. In the present invention, the measurement data of such a known sample is used as training data for Al training in the following manner. FIG. 6 shows the measurement results of a known sample with a known concentration of a target antigen, but in the present invention, the Al may also be trained using a known sample with a known presence or absence of a target antigen, a known sample with a known concentration of a microorganism, or a known sample with a known presence or absence of a microorganism.

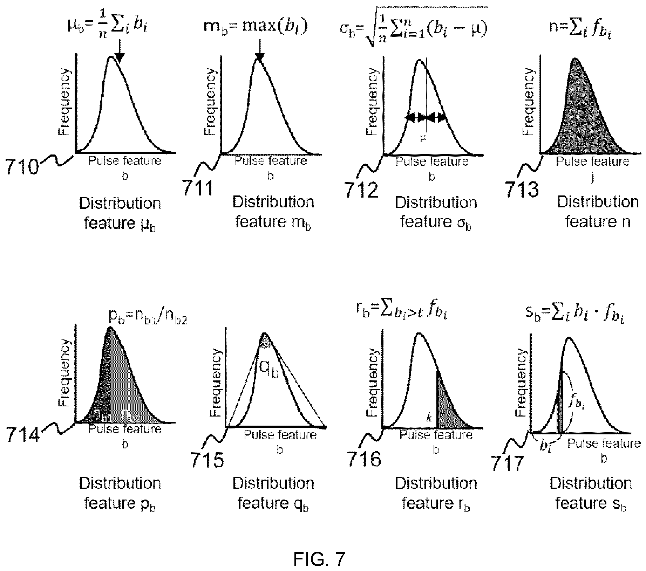

For each pulse waveform group, a distribution feature expressing the characteristics of the distribution of pulse feature such as those shown in FIG. 6 can be calculated. FIG. 7 shows an example of the distribution feature for pulse feature b. Examples include the mean µ b 710, mode m b 711, and standard deviation σ b 712 of pulse feature b. In addition, the number of pulses n 713 in the pulse waveform group is also a distribution feature. Other examples of distribution features 714 to 717 that indicate the characteristics of the histogram shape are also examples of distribution features of pulse feature b. The distribution feature in the present invention is not limited to that shown in FIG. 7, and may be any feature that expresses the characteristics of the distribution of pulse features. In addition, FIG. 7 illustrates the distribution feature of the pulse feature b, but various distribution features can be defined for each pulse feature. In the present invention, the distribution feature or both the distribution feature and the pulse feature can be used to train the Al model.

The Al algorithm to be trained in the present invention may be of any type. For example, the learning method may be a support vector machine, a linear discriminant transform, a k-nearest neighbor method, a decision tree, or an ensemble learning of these, or various deep learning methods, a recursive algorithm, a Boltzmann machine, or the like, but is not limited to these. When investigating whether or not an unknown sample contains a target antigen or target microorganism, a classification algorithm that outputs a binary value may be used, while when quantifying, a regression algorithm that outputs a continuous amount may be used. In addition, when identifying the type of target antigen or target microorganism, an Al algorithm that outputs a multi-value classification result of the number of types may be used.

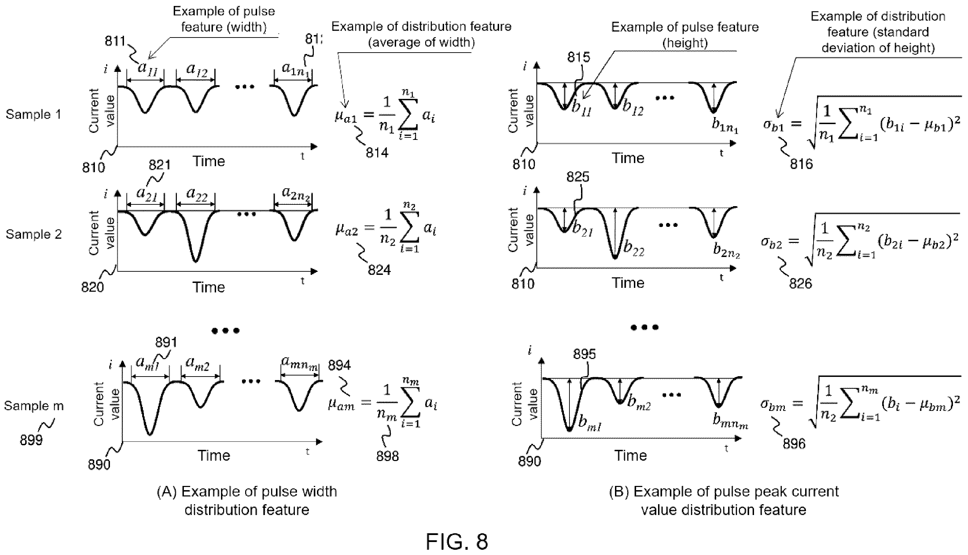

Al training using pulse features and distribution features according to the present invention will be described with reference to FIG. 8. Assume that m measurements are performed on samples 1 to m to provide teacher data for Al training. For each sample, ni (i is the sample number) pulse waveforms are measured. For example, in FIG. 8A, n1 pulse features a₁ⱼ (j is a pulse number) of pulse widths a₁₁ (811) to a₁ₙ₁ (812) are obtained as examples of pulse features for each pulse in the measurement result 810 of the sample 1. The average of these becomes the distribution feature µₐ₁ (814) which is the average of the pulse feature of the pulse width a in the sample 1. Similarly, m distribution features µₐᵢ (i is the sample number) which are the average of the pulse width a can be created for all measurements. In the present invention, for example, an Al model can be trained using these distribution features µₐᵢ as training data, and the target antigen concentration of sample i, the presence or absence of target antigen, the presence or absence of microorganisms, the concentration of microorganisms, etc. as correct answer labels. In addition, for example, FIG. 8B shows a schematic diagram of a method for calculating a distribution feature, which is a standard deviation σ_{bi} within a pulse waveform group, from a peak current bᵢⱼ, which is a pulse feature, from the measurement data of a sample i. Similarly, the Al model can be trained using bᵢⱼ as training data, with the target antigen concentration of sample i, the presence or absence of target antigen, the presence or absence of microorganisms, the concentration of microorganisms, etc. as correct answer labels.

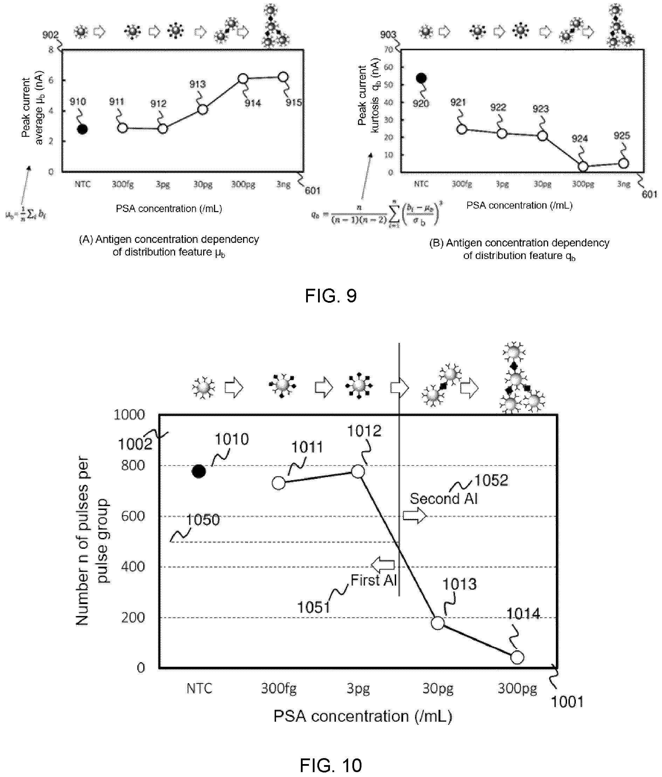

FIG. 9 shows the PSA concentration dependency of the distribution feature of the pulse feature b (pulse peak current) shown in FIG. 6. FIG. 9A shows the average of the pulse feature b, and 910 to 915 respectively denote the distribution feature µ_{b} that expresses the distribution of the pulse features shown as 610 to 615 in FIG. 6. FIG. 9B shows another example of the distribution feature. This is the kurtosis q of the distribution of the pulse feature expressed by the following equation, and 920 to 925 are the distribution features q_{b} of 610 to 615 in FIG. 6, respectively.
${q}_{bj} = \frac{{n}_{j}}{\left({n}_{j}-1\right) \left({n}_{j}-2\right)} {{\sum }_{i = 1}^{{n}_{j}} \left(\frac{{b}_{ji} - {μ}_{bj}}{{σ}_{bj}}\right)}^{3}$ wherein, n is the number of pulses, j is the pulse group number, i is the pulse number within pulse group j, and nⱼ is the total number of pulses within pulse group j. That is, distribution feature q expresses the kurtosis of the distribution of pulse feature b in pulse group j. As can be seen from Fig. 9, these distribution features well reflect the PSA concentration, and it can be seen that highly accurate PSA quantification is possible by training an Al using these distribution features as teacher data and the PSA concentration as a teacher label. In the present invention, an Al model can be trained using a plurality of distribution features, such as µ and q, as training data. This makes it possible to more accurately reflect the properties of a known sample that cannot be reflected by a single distribution feature in the Al model. In the example of FIG. 9, a plurality of distribution features are derived from one pulse waveform feature, namely, peak current b. However, a plurality of distribution features can also be calculated from, for example, pulse width a. In the present invention, a plurality of types of distribution features may be calculated from a plurality of types of pulse waveform features, and these may be used as teaching data to train the Al.

FIG. 10 shows the PSA concentration 1001 dependency of the number of pulses n 1002 per pulse group for the measurement exemplified in FIG. 6. Plots 1010 to 1014 correspond to plots 610 to 614 in FIG. 6. Since the number of pulses is the sum of the frequency distributions shown in FIG. 6, the number of pulses n is also one of the distribution features, and is illustrated as distribution feature n713 in FIG. 7. It can be seen that this distribution feature n also reflects the state of particle aggregation.

The number of distribution features that can be created is the product of the number of pulse waveform groups and the combination of the number of pulse features and the number of distribution features. In the present invention, the distribution feature that best expresses the state of the antibody-modified beads in the measurement target sample is used to detect and quantify the target antigens and target microorganisms in the measurement target sample.

So far, as one example of an embodiment of the present invention, a method for measuring the surface state of antibody-modified particles due to a target antigen or the aggregation state between antibody-modified particles by the method according to the present invention and further analyzing the data as shown in FIG. 4 has been described. As another example embodiment of the present invention, detection and quantification may be performed using multiple trained Al models based on the distribution feature. In the example of FIG. 4B, when the concentration of the target antigen is low, the adhesion state of the target antigen to the antibody-modified particle surface is read out as pulse waveforms 441 to 443. On the other hand, in the example of FIG. 4C, when the concentration of the target antigen is high, the agglutination state of the antibody-modified particles is read out in the pulse waveforms 461 to 463. Since the phenomena that are the subject of training are different, namely, B is antigen attachment to antibody-modified particles and C is aggregation between antibody-modified particles, better performance can be expected by training different Al models for each of FIG. 4B and FIG. 4C.

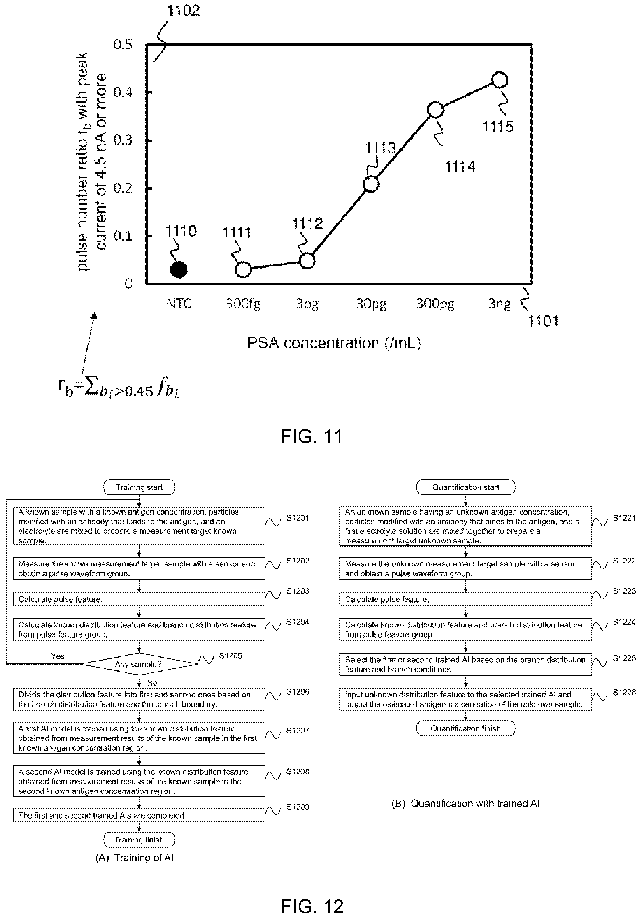

For example, the peak current kurtosis q_{b} shown in FIG 9B has a negative correlation with the PSA concentration from NTC to 30 pg/mL (920 to 923), and is a distribution feature suitable for estimating the target antigen concentration when the target antigen is dilute, as shown in FIG. 4B. However, this q_{b} does not have a negative correlation with PSA concentrations from 300 pg/mL to 3 ng/mL (924 and 925), and is not suitable for estimating the target antigen concentration in a state such as that shown in FIG. 4C. On the other hand, in the case of the pulse number ratio r_{b} for a peak current of 4.5 nA or more, which is another distribution feature shown in FIG. 11, 30 pg/mL to 3 ng/mL (1113 to 1115) is positively correlated with the PSA concentration, and it can be seen that in the target antigen concentration region of FIG. 4C, this distribution feature is more suitable for estimating the target antigen concentration than the above q_{b}.

Therefore, in one example of the present invention, the distribution feature n shown in FIG. 10 is used for branching in Al training. Such a distribution feature used as a branch condition is called a branch distribution feature. Here, the distribution feature n is the branch distribution feature, and the number n = 500 is the branch condition 1050. In this example, a pulse waveform group 1051 where n < 500 is used for training the first Al as the region represented in FIG 4A and FIG. 4B. The first Al will be trained with a model that places greater weight on the distribution feature q_{b} 903 for the target antigen concentration, which is the teacher label. On the other hand, the pulse waveform group 1052 where n ≥ 500 is used for training the second Al as the region shown in FIG. C. In the second AI, training will be performed using a model that places greater weight on the distribution feature r_{b} 1102 for the target antigen concentration, which is the teacher label. In the quantification of an unknown sample, if the distribution feature n is n < 500, the distribution feature is input to the first Al, which outputs an estimated value of the target antigen concentration. If n ≥ 500, the distribution feature is input to the second Al, which outputs an estimated value of the target antigen. In the present invention, by dividing the differences in particle states that are the subject of training based on distribution features and training multiple Al models, it is possible to provide higher performance. As in the example of Figure 4, when the particle state changes qualitatively in response to changes in the target antigen concentration or the like, this embodiment, in which branch boundaries are set and a plurality of Als are trained, is effective. In this example, one branch feature is used as the branch condition, but a plurality of distribution features may be used as the branch condition. In addition, in this example, the branch condition is divided into two regions with the branch distribution feature n as the boundary, but the branch condition may be configured with logic that improves the results of detection and quantification. Furthermore, in the present invention, a known antigen concentration may be used as a branch condition during Al training, and a branching distribution feature may be used as a branch condition during quantification.

One embodiment of the present invention for training such a plurality of Al models is illustrated in FIG. 12. FIG. 12A shows Al training for quantification. First, a known sample with a known antigen concentration and a known measurement target sample containing particles modified with an antibody that binds to the target antigen and an electrolyte are prepared (step S1201). Next, this is measured by a sensor such as that shown in FIG. 1, and a known pulse waveform group consisting of a plurality of pulses is measured (step S1202). A known pulse feature of each pulse waveform in the known pulse waveform group is calculated (step S1203), and then a known distribution feature is calculated from the known pulse features (step S1204). In this example of quantification, any number of types of known pulse features may be used, and any number of types of distribution features may be calculated from each of the plurality of known pulse features. Among the calculated known distribution features, the distribution features to be used for branching in the Al learning (hereinafter referred to as branch distribution feature) are determined. Any number of branch distribution features may be used among the known distribution features. Further, a distribution feature not used for Al training can be used as a branch distribution feature. Steps S1201 to S1204 are performed for all samples used for training as teacher data. In the above example, the branch distribution feature is n. For one sample (for example, sample number m899 in FIG. 8), a pulse waveform group consisting of a plurality of pulse waveforms (for example, pulse number nₘ 898 in FIG. 8) and one or more distribution features (for example, µₐₘ 894 and σ_{bm} 896 in FIG. 8) can be obtained.

A sample to be used as training data is measured, and the known distribution feature and branch distribution feature are calculated for each pulse waveform group (step S1205). Then, the branch feature is divided into pulse distribution feature that is above the branch boundary and pulse distribution feature that is below the branch boundary (step 1206). The first Al model is trained (step S1207) using the former known distribution feature as training data and the corresponding known antigen concentrations as training labels. In addition, a second AI model is trained using the latter known distribution feature as training data and the corresponding known antigen concentrations as training labels (step S1207).

After training the first and second AI models in this manner, the present invention performs quantification of target antigens in unknown samples using a method such as that shown in FIG. 12B. First, an unknown measurement target sample is prepared, which contains an unknown sample with an unknown antigen concentration, particles modified with an antibody that binds to the target antigen, and a first electrolyte solution (step S1221). Next, this is measured by a sensor such as that shown in FIG. 1, and an unknown pulse waveform group consisting of a plurality of pulses is measured (step S1222). An unknown pulse feature for each pulse waveform in the unknown pulse waveform group is calculated (step S1223), and then an unknown distribution feature and a branch feature are calculated from the unknown pulse feature (step S1224). Then, using the branch distribution feature, it is selected whether the first or second trained Al is to be used for quantifying the unknown sample (step S1225).The unknown distribution feature is then input to the selected trained Al, and the antigen concentration estimation result of the unknown sample is output.

In this embodiment of the present invention, for the phenomenon in which the manner in which particles pass through pores changes qualitatively depending on the antigen concentration, etc., different Al models are individually trained to suit each of the different modes, making it possible to train AI with higher accuracy.

In the examples of FIGS. 5, 6, 9, 10 and 11, the pulse waveform when an antibody-modified particle passes through a pore has been described as the subject of the data analysis method of the present invention, but in the present invention, the pulse waveform when a biological particle such as a bacterial particle, a virus particle, or an extracellular endoplasmic reticulum passes through a pore may also be analyzed using a similar method.

The method according to the present invention as described above can be implemented by a computer device or terminal having a processor (which may be a single processor or a multi-core processor, and may also include a microprocessor). Such a computing device may be a device integrated with a sensor, such as sensor 100 of FIG. 1. Alternatively, it may be a computer device that is operatively connected to such a sensor via a network (whether wired or wireless, such as the Internet, a dedicated closed network, or a LAN).

The computer device that can be used in relation to the present invention may take any form, such as a workstation, a tablet, a smartphone, and the like.

In an embodiment of the present invention, a computer readable program may be provided, which, when executed by a processor, may cause any of the steps of the methods described above to be performed.

### EXAMPLES

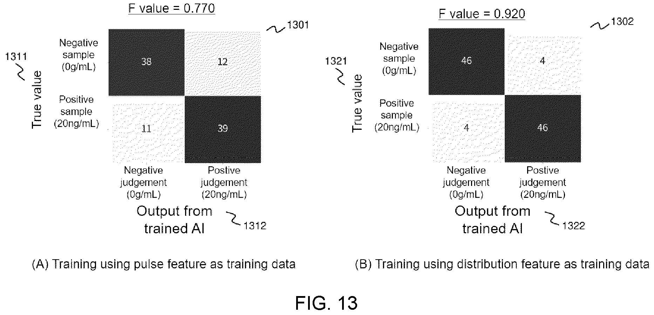

In order to demonstrate an example of the effect of the present invention, the following experiment was carried out using influenza N protein as the target antigen. First, 50 negative samples were prepared by mixing and incubating a sample not containing the target antigen with an electrolyte solution containing dispersed 80 nm polystyrene beads whose surfaces were modified with anti-influenza N protein antibodies, and 50 positive samples were prepared by mixing and incubating a sample containing the target antigen at a concentration of 20 ng/mL. The Al was then trained by measuring each sample using the method described above. FIG. 13 is a confusion matrix for evaluating the performance of a trained Al by cross-validation. Vertical 1311 and 1321 are the true positive and negative values of known samples, and horizontal 1312 and 1322 are the Al outputs of the trained Al estimating positive and negative values. The diagonal line sloping downward to the right indicates the number of correct answers. Fig. 13A shows the performance of a trained Al model trained using pulse features as teacher data by the conventional method, and FIG. 13B shows the performance of a trained Al model trained using distribution features as teacher data by the method of the present invention. The F-value of the conventional method was 0.770, whereas the F-value of the method of the present invention using the distribution feature as training data was 0.920, which shows a significant improvement in performance.

## Claims

1. A method for preparing a data for estimating a concentration of an unknown antigen in an unknown sample in which a concentration of a target antigen is unknown; the method using a sensor comprising two chambers separated by a partition wall and communicating with each other through a pore, and an electrode in each of the two chambers; the method comprising:
a step of preparing a first sensor comprising two chambers configured to be electrically connected via a first pore and an electrolyte, and placing a known measurement target sample, which includes a known sample containing the target antigen with a known antigen concentration and an antibody-modified particle whose surface is modified with an antibody that specifically binds to the target antigen, into one of the chambers of the first sensor;
a step of applying a voltage between the electrodes of each chamber of the first sensor to cause an ionic current to flow between the two electrodes of the first sensor via the first pore, and measuring a transient change in the ionic current caused by the antibody-modified particles in the known measurement target sample passing through the first pore as a known pulse waveform group consisting of a plurality of known pulse waveforms;
a step of calculating a known pulse waveform feature that expresses a shape characteristic for each of the plurality of known pulse waveforms belonging to the known pulse waveform group;
a step of calculating a known distribution feature that expresses a distribution characteristic of the known pulse waveform feature within the known pulse waveform group; and
a step of training an Al model using the known distribution feature as a training data and the known antigen concentration as a training label to create a trained Al model.

2. The method according to claim 1, wherein the method further comprises:
a step of preparing a second sensor having two chambers configured to be electrically connected via a second pore and an electrolyte, and placing unknown measurement target sample, which includes an unknown sample containing the target antigen with an unknown concentration and the antibody-modified particles, into one of the chambers of the second sensor;
a step of applying a voltage between the electrodes of each chamber of the second sensor to cause an ionic current to flow between the two electrodes of the second sensor via the second pore, and measuring a transient change in the ionic current caused by the antibody-modified particles in the unknown measurement target sample passing through the second pore as an unknown pulse waveform group consisting of a plurality of unknown pulse waveforms;
a step of calculating an unknown pulse waveform feature that expresses a shape characteristic for each of the unknown pulse waveforms belonging to the unknown pulse waveform group;
a step of calculating an unknown distribution feature that expresses a distribution characteristic of the unknown pulse waveform feature within the unknown pulse waveform group; and
a step of inputting the unknown distribution feature into the trained Al model, and causing the trained Al model to output an estimate of the unknown antigen concentration.

3. The method according to claim 1, wherein the method further comprises a step of:
calculating a known branch distribution feature from the known pulse waveform feature belonging to the known pulse waveform group; and
if the known branch distribution feature satisfies a branch condition, training a first Al using the known distribution feature as a training data and the known antigen concentration as a training label to create a first trained AI, or
if the known branch distribution feature does not satisfy the branch condition, training a second AI using the known distribution feature as a training data and the known antigen concentration as a training label to create a second trained AI.

4. The method according to claim 3, wherein the method further comprises:
a step of preparing a second sensor comprising two chambers configured to be electrically connected via a second pore and an electrolyte, and placing an unknown measurement target sample, which includes an unknown sample containing the target antigen with unknown concentration and the antibody-modified particles, into one of the chambers of the second sensor;
a step of applying a voltage between the electrodes of each chamber of the second sensor to cause an ionic current to flow between the two electrodes of the second sensor via the second pore, and measuring a transient change in the ionic current caused by the antibody-modified particles in the unknown measurement target sample passing through the second pore as an unknown pulse waveform group consisting of a plurality of unknown pulse waveforms;
a step of calculating an unknown pulse waveform feature that expresses a shape characteristic for each of the unknown pulse waveforms belonging to the unknown pulse waveform group;
a step of calculating an unknown branch distribution feature that expresses a distribution characteristic of the unknown pulse waveform feature within the unknown pulse waveform group; and
a step of calculating an unknown branch distribution feature from the unknown pulse waveform feature belonging to the unknown pulse waveform group, and
if the unknown branch distribution feature satisfies the distribution condition, inputting the unknown distribution feature to the first trained Al, and outputting an estimated value of the unknown antigen concentration, or
if the unknown branch distribution feature does not satisfy the distribution condition, inputting the unknown distribution feature to the second trained AI and outputting an estimate of the unknown antigen concentration.

5. The method according to claim 1, wherein the method further comprises a step of:
if the known antigen concentration satisfies a branch condition, training a first Al using the known distribution feature as a training data and the known antigen concentration as a training label to create a first trained Al, or
If the known antigen concentration does not satisfy the branch condition, training a second AI using the known distribution feature as a training data and the known antigen concentration as a training label to create a second trained AI.

6. A method for preparing a data to estimate whether a target microparticle is present in an unknown sample, the method using a sensor comprising two chambers separated by a partition wall and communicating with each other through a pore, and an electrode in each of the two chambers; the method comprising:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor;
a step of applying a voltage between electrodes of each of the chambers of the first sensor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms;
a step of calculating a known pulse waveform feature that expresses a shape characteristic for each of the plurality of known pulse waveforms;
a step of calculating a known distribution feature that expresses a distribution characteristic of the known pulse waveform feature within the known pulse waveform group; and
a step of training an Al model using the distribution feature as a training data and information expressing the presence or absence of the target particle in the known sample as a training label to create a trained Al model.

7. The method according to claim 6, wherein the method further comprises:
a step of preparing a second sensor having two chambers configured to be electrically connected via a second pore and an electrolyte, and placing an unknown measurement target sample, which includes an unknown sample in which the presence or absence of the target microparticle is unknown and an electrolyte, into one of the chambers of the second sensor;
a step of applying a voltage between electrodes of each of the chambers of the second sensor;
a step of measuring a transient change in an ion current caused by a microparticle contained in the unknown measurement target sample passing through the second pore while an ion current is caused to flow between two electrodes of the second sensor via the second pore, as an unknown pulse waveform group belonging to the unknown measurement target sample, the unknown pulse waveform group consisting of a plurality of unknown pulse waveforms;
a step of calculating an unknown pulse waveform feature that expresses a shape characteristic for each of the plurality of unknown pulse waveforms;
a step of calculating an unknown distribution feature that expresses a distribution characteristic of the unknown pulse waveform feature within the unknown pulse waveform group; and
a step of inputting the unknown distribution feature into the trained Al model to estimate whether the target microparticle is contained in the unknown sample.

8. The method according to claim 6, wherein the target microparticle is a bacteria.

9. The method according to claim 6, wherein the target microparticle is a virus.

10. A method for preparing a data to estimate whether a target microparticle is present in an unknown sample, the method using a sensor comprising two chambers separated by a partition wall and communicating with each other through a pore, and an electrode in each of the two chambers; the method comprising:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor;
a step of applying a voltage between electrodes of each of the chambers of the first sensor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms;
a step of c calculating a known pulse waveform feature that expresses a shape characteristic for each of the known pulse waveforms; and
a step of calculating a known distribution feature that expresses a distribution characteristic of the known pulse waveform feature within the known pulse waveform group; and
a step of training an Al model using the known pulse waveform feature and the known distribution feature as training data and information expressing the presence or absence of the target particle in the known sample as training labels to create a trained Al model.

11. The method according to claim 1, 6, or 10, wherein the known pulse waveform feature is calculated from an output of a convolutional neural network.

12. A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a first sensor comprising two chambers configured to be electrically connected via a first pore and an electrolyte, and placing a known measurement target sample, which includes a known sample containing the target antigen with a known antigen concentration and an antibody-modified particle whose surface is modified with an antibody that specifically binds to the target antigen, into one of the chambers of the first sensor, by the processor;
a step of applying a voltage between the electrodes of each chamber of the first sensor to cause an ionic current to flow between the two electrodes of the first sensor via the first pore, and measuring a transient change in the ionic current caused by the antibody-modified particles in the known measurement target sample passing through the first pore as a known pulse waveform group consisting of a plurality of known pulse waveforms, by the processor;
a step of calculating a known pulse waveform feature that expresses a shape characteristic for each of the plurality of known pulse waveforms belonging to the known pulse waveform group, by the processor;
a step of calculating a known distribution feature that expresses a distribution characteristic of the known pulse waveform feature within the known pulse waveform group, by the processor; and
a step of training an Al model using the known distribution feature as a training data and the known antigen concentration as a training label to create a trained Al model, by the processor.

13. A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor, by the processor;
a step of applying a voltage between electrodes of each of the chambers of the first sensor, by the processor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms, by the processor;
a step of calculating a known pulse waveform feature that expresses a shape characteristic for each of the plurality of known pulse waveforms, by the processor;
a step of calculating a known distribution feature that expresses a distribution characteristic of the known pulse waveform feature within the known pulse waveform group, by the processor; and
a step of training an Al model using the distribution feature as a training data and information expressing the presence or absence of the target particle in the known sample as a training label to create a trained Al model, by the processor.

14. A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform the steps of:
a step of preparing a first sensor comprising two chambers configured to be electrically connected via a first pore and an electrolyte, and placing a known measurement target sample, which includes a known sample in which the presence or absence of the target microparticle is known and an electrolyte, into one of the chambers of the first sensor, by the processor;
a step of applying a voltage between electrodes of each of the chambers of the first sensor, by the processor;
a step of measuring a transient change in an ion current caused by a microparticle contained in the known measurement target sample passing through the first pore while an ion current is caused to flow between two electrodes of the first sensor via the first pore, as a known pulse waveform group belonging to the known measurement target sample, the known pulse waveform group consisting of a plurality of known pulse waveforms, by the processor;
a step of calculating a known pulse waveform feature that expresses a shape characteristic for each of the known pulse waveforms, by the processor; and
a step of calculating a known distribution feature that expresses a distribution characteristic of the known pulse waveform feature within the known pulse waveform group, by the processor; and
a step of training an Al model using the known pulse waveform feature and the known distribution feature as training data and information expressing the presence or absence of the target particle in the known sample as training labels to create a trained Al model, by the processor.

15. The method according to claim 1, 6 or 10, or the program according to any one of claims 12 to 14, wherein the antigen is replaced with an antibody and the antibody is replaced with an antigen.
